Europäisches Patentamt

European Patent Office

Office européen des brevets

(19)

(11) Publication number: **0 276 462 B1**

# (12) EUROPEAN PATENT SPECIFICATION

(45) Date of publication of patent specification: **16.09.92**   (51) Int. Cl.5: **B60K 28/06**, //G08B21/00

(21) Application number: **87118971.8**

(22) Date of filing: **21.12.87**

(54) **Acoustic sleep inhibiting device.**

(30) Priority: **19.12.86 IT 6794886**

(43) Date of publication of application:
**03.08.88 Bulletin  88/31**

(45) Publication of the grant of the patent:
**16.09.92 Bulletin  92/38**

(84) Designated Contracting States:
**DE ES FR GB SE**

(56) References cited:

PATENT ABSTRACTS OF JAPAN, vol. 9, no.
213 (M-408)[1936], 30th August 1985, & JP-
A-60 71 338 (OKI DENKI KOGYO K.K.)
23-04-1985

PATENT ABSTRACTS OF JAPAN, vol. 9, no.
202 (M-405)[1925], 20th August 1985; & JP-
A-60 64 038 (SUZUKI JIDOSHA KOGYO K.K.)
12-04-1985

PATENT ABSTRACTS OF JAPAN, vol. 8, no.
267 (M-343)[1704], 7th December 1984; & JP-
A-59 140 134 (SEIKOUSHIYA K.K.) 11-08-1984

PATENT ABSTRACTS OF JAPAN, vol. 6, no.
267 (M-182)[1145], 25th December 1982; &

JP-A-57 158 126 (MITSUBISHI DENKI K.K.)
29-09-1982

(73) Proprietor: **FIAT AUTO S.p.A.**
**Corso Giovanni Agnelli 200**
**I-10135 Torino(IT)**

(72) Inventor: **Fubini, Enrica**
**Via Viberti, 23**
**I-10100 Torino(IT)**
Inventor: **Ruspa, Giacomo**
**Viale Giacomo Matteotti, 4**
**I-10048 Vinovo(IT)**
Inventor: **De Bono, Antonio**
**Via Caraglio, 49**
**I-10100 Torino(IT)**

(74) Representative: **Prato, Roberto et al**
**STUDIO TORTA Società Semplice Via Viotti 9**
**I-10121 Torino(IT)**

## Description

The present invention relates to an acoustic sleep inhibiting device, particularly, though not exclusively, suited for fitment to motor vehicles, for keeping the driver alert.

Numerous devices are available for fitment to motor vehicles, for safeguarding the driver against drowsing at the wheel. Such devices usually comprise sensors appropriately located on the steering wheel, the nape of the neck, eyelids, etc., for continuously monitoring a given parameter (e.g. hand pressure on the steering wheel blinking of the eyelids, etc.) and indicating, e.g. by means of a sound alarm, any reduction in concentration on the part of the driver.

Though widely used, a major drawback of devices of the aforementioned type is that they often operate too late, i.e. when the situation has already become critical. The aim of the present invention is to provide an acoustic sleep inhibiting device designed to maintain a certain level of concentration on the part of the driver, and so overcome the drawbacks associated with the aforementioned known types of devices.

With this aim in view, according to the present invention, there is provided a sleep inhibiting device, conveniently designed for fitment to motor vehicles, for retaining the attention of the driver; characterised by the fact that it comprises:

- a generator for generating sound messages having complex sound characteristics evolving in non-periodical manner; and
- means for enabling the said generator, which means may be activated voluntarily or involuntarily and indirectly by the driver.

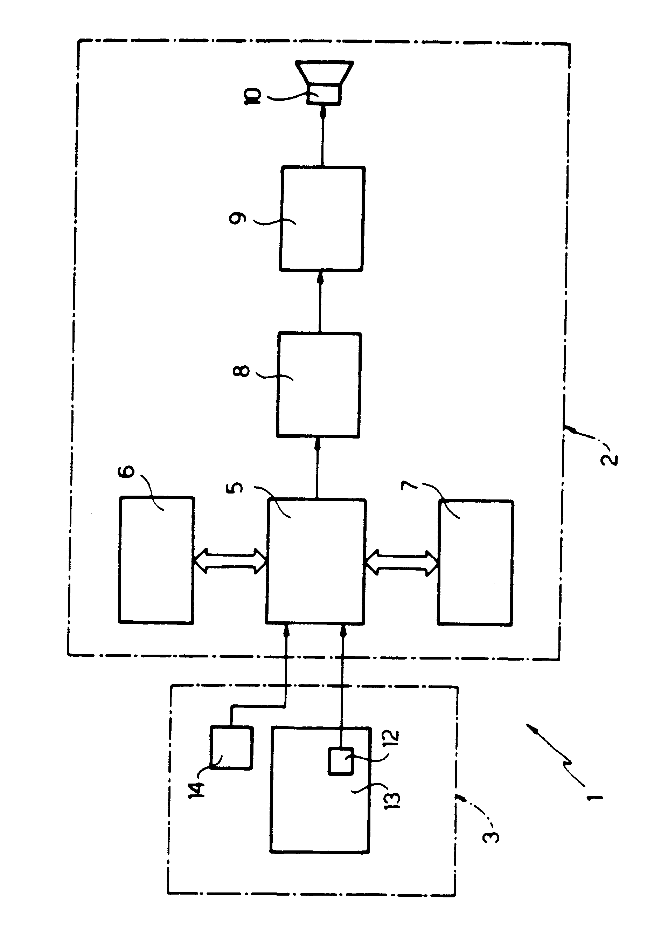

A preferred embodiment of the present invention will be described by way of a non-limiting example with reference to the attached drawing, which shows an operating block diagram of the same.

Number 1 in the attached block diagram indicates a sleep inhibiting device according to the present invention and substantially comprising a generator 2 for generating sound messages, and means 3 for enabling the said generator 2.

Generator 2 conveniently consists of a processing unit 5 comprising, for example, a microprocessor interfacing with a non-volatile (e.g. ROM) memory 6 and a random signal generator 7.

Memory 6 conveniently contains data relative to a number of messages. The data relative to each message is grouped together and connected to a single address selectable by processing unit 5 on the basis of signals supplied by generator 7.

Message generator 2 also comprises a signal synthesizer 8 having its input connected to processing unit 5 and its output connected to a loudspeaker 10 via amplifier 9.

Means 3 for enabling message generator 2 conveniently comprise a key 12, which may be operated by the driver and is located on the vehicle dashpanel 13, and a known type of sensor 14 for detecting a parameter depending on the concentration level of the driver. Key 12 and sensor 14 are connected to enabling inputs of processing unit 5.

Device 1 operates as follows. Upon feling drowsy, the driver presses key 12 which enables processing unit 5. On the basis of random signals generated continually by generator 7, processing unit 5 withdraws from memory 6 data relative to various sound messages. The said data is then sent by processing unit 5 to signal synthesizer 8 which, in turn, sends it to loudspeaker 10 via amplifier 9. By virtue of generator 7, the sound messages transmitted by loudspeaker 10 evolve in non-repetitive manner and may be separated by time intervals which are also irregular. The said sound messages may conveniently consist of phrases or various types of acoustic signals having complex sound characteristics, the intensity, height and tone of which may also vary in random manner.

The advantages of the device according to the present invention will be clear from the foregoing description. First and foremost, it may be operated directly and voluntarily by the driver, who is subjected to a sequence of irregularly-spaced sound messages having complex sound characteristics evolving in non-periodical manner, which, by creating a feeling of expectancy in the driver, contribute towards maintaining the driver's attention.

A single alarm signal is therefore replaced by a continuous supply of sound messages, the sleep inhibiting effect of which is undoubtedly far more effective than a single, and invariably tardy, alarm signal. In the event of drowsiness on the part of the driver, and failure of the same to activate the device voluntarily, means 3 for enabling sensor 14 activate generator 2 automatically.

To those skilled in the art it will be clear that changes may be made to device 1 as described herein without, however, departing from the scope of the present invention.

## Claims

1. A sleep inhibiting device designed for fitment to motor vehicles, for retaining the attention of the driver, comprising a sound generator (2) for generating sound messages having complex sound characteristics, and enabling means (3) activatable voluntary or involuntary for enabling said sound generator (2), said sound generator (2) including memory means (6) storing data relative to said sound messages and an electroacustic transducing means (10) controlled by a processing unit (5) according to the data stored in said memory means (6), characterised in that said memory means (6) stores data relative to a number of messages, the data relative to each of said messages being grouped together to a single address of said memory means (6), a continuously operating random generator (7) being provided for generating random signals conditioning said processing unit (5) when so activated to randomly address the data of said memory means (6), whereby said messages are produced and evolve in a non-periodical and non-repetitive manner.

2. A device as claimed in claim 1, characterised in that said sound generator (2) also include a sound synthesizer (8) connected to said transducing means (10), said sound messages including phrases.

3. A device as claimed in claim 1 or 2, characterised in that said transducing means include a loud speaker (10), said sound messages being of randomly varying intensity, height and tone.

4. A device as claimed in claim 3, characterised in that said processing unit includes a microprocessor (5) interfacing said memory means (6), said synthesizer (8) and said random generator (7).

5. A device as claimed in claim 4, characterised in that said enabling means include a manually depressible key (12) located on a vehicle panel (13) and a sensor (14) for detecting a parameter depending on the concentration level of the driver, said key (12) and said sensor (14) being connected to corresponding enabling inputs of said microprocessor (5).

## Patentansprüche

1. Schlafhemmungsvorrichtung, die für den Einbau in ein motorisiertes Fahrzeug ausgebildet ist, um die Aufmerksamkeit des Fahrers zu erhalten, welche einen Schallgenerator (2) zum Erzeugen akustischer Nachrichten mit komplexen akustischen Merkmalen, und Einschaltmittel (3), welche freiwillig oder unfreiwillig zum Einschalten des Schallgenerators (2) aktivierbar sind, aufweist, wobei der Schallgenerator (2) Speichermittel, welche sich auf die akustischen Nachrichten beziehenden Daten speichert, und elektroakustische Übertragungsmittel (10), die von einer Prozessoreinheit (5) entsprechend den in den Speichermitteln (6) gespeicherten Daten gesteuert werden, enthält dadurch gekennzeichnet, daß die Speichermittel (6) Daten speichern, die sich auf eine Anzahl von Nachrichten beziehen, wobei die sich auf jede Nachricht beziehenden Daten Zu einer einzigen Adresse der Speichermittel (6) zusammengefaßt sind, wobei ein kontinuierlich arbeitender Zufallsgenerator (7) zur Erzeugung von Zufallssignalen, welche die Prozessoreinheit (5) bei Aktivierung konditionieren, um auf Zufallsbasis die Daten der Speichermittel (6) zu adressieren, vorgesehen ist, wodurch die Nachrichten in einer nichtperiodischen und sich nicht wiederholenden Weise erzeugt werden und sich entwickeln.

2. Vorrichtung nach Anspruch 1, dadurch gekennzeichnet, daß der Schallgenerator (2) ferner eine schallsynthetisierende Vorrichtung (8) aufweist, welche mit den Übertragungsmitteln (10) verbunden ist, wobei die akustischen Nachrichten Phrasen beinhalten.

3. Vorrichtung nach einem der Ansprüche 1 oder 2, dadurch gekennzeichnet, daß die Übertragungsmittel einen Lautsprecher (10) aufweisen, wobei die akustischen Nachrichten von zufällig variierender Intensität, variierender Höhe und variierendem Klang sind.

4. Vorrichtung nach Anspruch 3, dadurch gekennzeichnet, daß die Prozessoreinheit einen Mikroprozessor (5) aufweist, der mit den Speichermitteln (6), dem Synthetisierer (8) und dem Zufallsgenerator (7) Schnittstellen bildet.

5. Vorrichtung nach Anspruch 4, dadurch gekennzeichnet, daß die Einschaltmittel einen manuell drückbaren Taste (12), die auf dem Fahrzeugamaturenbrett (13) angeordnet ist, und einen Sensor (14) zur Feststellung eines vom Konzentrationspegel des Fahrers abhängigen Parameters aufweisen, wobei der Schlüssel (12) und der Sensor (14) mit entsprechenden Einschalteingängen des Mikroprozessors (5) verbunden sind.

## Revendications

1. Dispositif empêchant la somnolence, conçu pour être monté sur des véhicules automobiles pour maintenir l'attention du conducteur, comprenant un générateur acoustique (2) pour produire des messages acoustiques présentant des caractéristiques acoustiques complexes, et des moyens de validation (3) pouvant être activés volontairement ou involontairement pour valider ledit générateur acoustique (2), ledit générateur acoustique (2) comprenant des moyens de mémoire (6) mémorisant des données relatives auxdits messages acoustiques, et des moyens de transduction électroacoustique (10) commandés par une unité de traitement (5) en fonction des données mémorisées dans lesdits moyens de mémoire moire (6), caractérisé en ce que lesdits moyens de mémoire (6) mémorisent les données relatives à un nombre de messages, les données relatives à chacun desdits messages étant regroupées en une seule adresse desdits moyens de mémoire (6), un générateur de signaux aléatoires (7) fonctionnant en continu étant prévu pour produire des signaux aléatoires conditionnant ladite unité de traitement (5), lorsqu'il est ainsi activé, pour adresser de façon aléatoire les données desdits moyens de mémoire (6), ce qui provoque la production et la délivrance desdits messages d'une manière non périodique et non répétitive.

2. Dispositif selon la revendication 1, caractérisé en ce que ledit générateur acoustique (2) comprend également un synthétiseur acoustique (8) raccordé auxdits moyens de transduction (10), lesdits messages acoustiques comprenant des phrases.

3. Dispositif selon la revendication 1 ou 2, caractérisé en ce que lesdits moyens de transduction comprennent un haut-parleur (10), lesdits messages acoustiques possédant une intensité, une hauteur et une tonalité variant de façon aléatoire.

4. Dispositif selon la revendication 3, caractérisé en ce que ladite unité de traitement comprend un microprocesseur (5) raccordant lesdits moyens de mémoire (6), ledit synthétiseur (8) et ledit générateur de signaux aléatoires (7).

5. Dispositif selon la revendication 4, caractérisé en ce que lesdits moyens de validation comprennent une touche (12) pouvant être enfoncée manuellement, située sur un panneau (13) du véhicule, et un capteur (14) pour détecter un paramètre en fonction du niveau de concentration du conducteur, ladite touche (12) et ledit capteur (14) étant raccordés à des entrées de validation correspondantes dudit microprocesseur (5).